# EUROPEAN PATENT APPLICATION

(11) **EP 2 816 346 A1**
(43) Date of publication of application: **24.12.2014**
(21) Application number: 13754105.8
(22) Date of filing: 13.02.2013
(51) Int. Cl.: G01N 21/84

(54) **PILL NUMBER COUNTING DEVICE**

(30) Priority: 01.03.2012 JP 2012045965
(71) Applicant: Ookuma Electronic Co. Ltd., Kumamoto-shi, Kumamoto 861-8037 (JP)
(72) Inventor: OKUMA Keiji, Kumamoto-shi Kumamoto 861-8037 (JP)
(74) Representative: Jordan, Volker Otto Wilhelm
(86) International application number: PCT/JP2013/053325
(87) International publication number: WO 2013/129099

(57) **Abstract**

Pills in a dose packet having strings and/or drawing patterns printed or handwritten thereon are counted at high accuracy. A pill counting device counts pills in a dose packet including two near-infrared light transmissive sheets laminated and sealed at the periphery, a first sheet of the sheets having information identifiers thereon. The device includes an illuminator to emit near-infrared light toward the packet from below, while the first sheet faces downward; an imager disposed to receive the near-infrared light transmitted through the packet and take a digital image of the packet; a light quantity adjuster to adjust the quantity of the near-infrared light received by the imager; and a pill counter to binarize the image and count the pills. For a digital image of the packet containing no pill, the light quantity adjuster adjusts the quantity such that the gray scale values of substantially all the pixels exceed a threshold.

## Description

### Technical Field

The present invention relates to a pill counting device that automatically counts the number of pills enclosed in a dose packet.

### Background Art

Hospital pharmacies and dispensing pharmacies provide single dose packaging through enclosing a single dose of various pills for a patient into a single dose packet, for example, with a known automatic medicine packaging machine. In terms of providing proper pills to patients in accordance with a prescription, the counting operation of pills in each dose packet and the elimination of a dose packet containing an abnormal number of pills are essential.

A typical conventional pill inspecting machine automatically counts pills in a dose packet through the imaging of the dose packet with a CCD camera and the analysis of the taken image.

Dose packets often have strings (e.g., the name of a patient, the names of pills, and dates) and various drawing patterns thereon. Unfortunately, the images including the strings and drawing patterns disturb the counting operation of pills.

In order to solve this problem, various techniques have been disclosed (for example, refer to PTL 1).

### Citation List

### Patent Literature

PTL 1: Japanese Unexamined Patent Application Publication No. H7-209196

### Summary of Invention

### Technical Problem

PTL 1 discloses a pill inspecting system to determine the number or type of pills in a dose packet, for a pill packaging machine including a printing mechanism to print data on a surface of a light-transmissive dose packet and a packaging mechanism to enclose multiple pills into the dose packet. The pill inspecting system includes an imaging means to take an original image of a dose packet disposed at a position of inspection, a first image processing means to convert the original image taken by the imaging means into a first binary image, a second image processing means to acquire information on the print from the printing mechanism and convert the print information into a second binary image, a third image processing means to generate a third binary image through the subtraction of the second binary image from the first binary image, a fourth image processing means to generate a fourth binary image through the removal of the noise involving the contraction and expansion of the third binary image, and an image recognizing means to determine the number or type of pills in the dose packet through the image recognition on the fourth binary image.

The system disclosed in PTL 1 acquires print information from the printing mechanism, converts the print information into a second binary image, and subtracts the second binary image from a first binary image (binarized original image), to remove the image of the print on the dose packet. In clinical practice, for example, the number of the hospital ward and check marks such as ticks are often handwritten on a dose packet with an oil-based pen as required. Unfortunately, the system disclosed in PTL 1 cannot remove such handwritten information from the image. This configuration may reduce the accuracy of recognizing the image of pills in a dose packet having handwritten information thereon.

In addition, the system disclosed in PTL 1 needs to subtract the second binary image from the first binary image every counting of pills in a dose packet. This configuration may increase the processing load on the components, such as a controller, of the machine.

An object of the invention, which has been accomplished in view of the above conventional problems, is to provide a pill counting device that can count pills in a dose packet having strings and/or drawing patterns printed or handwritten thereon at high accuracy under reduced processing load on the device.

### Solution to Problem

A pill counting device according to a first aspect of the invention counts pills in a dose packet including two near-infrared light transmissive sheets laminated and sealed at the periphery, a first sheet of the sheets having an information identifier thereon. The device includes: an illuminator to emit near-infrared light toward the dose packet from below, the dose packet being disposed such that the first sheet faces downward; an imager disposed at a position to receive the near-infrared light transmitted through the dose packet, the imager taking a digital image of the dose packet; a light quantity adjuster to adjust the quantity of the near-infrared light received by the imager; and a pill counter to binarize the digital image and count the pills. For a digital image of the dose packet containing no pill, the light quantity adjuster adjusts the light quantity such that gray scale values of substantially all the pixels of the digital image exceed a predetermined threshold.

A specified pill counting device according to a second aspect is involved in the pill counting device according to the first aspect. In the specified pill counting device, the adjustment of the light quantity involves adjusting a luminance of the near-infrared light emitted from the illuminator.

A pill counting device according to a third aspect is involved in the pill counting device according to the first or second aspect. In the specified pill counting device, the adjustment of the light quantity involves adjusting an exposure time of the imager.

### Advantageous Effects of Invention

The present invention can provide a pill counting device for counting pills in a dose packet including two near-infrared light transmissive sheets laminated and sealed at the periphery, a first sheet of the sheets having an information identifier thereon, the device including: an illuminator to emit near-infrared light toward the dose packet from below, the dose packet being disposed such that the first sheet faces downward; an imager disposed at a position to receive the near-infrared light transmitted through the dose packet, the imager taking a digital image of the dose packet; a light quantity adjuster to adjust the quantity of the near-infrared light received by the imager; and a pill counter to binarize the digital image and count the pills, wherein for a digital image of the dose packet containing no pill, the light quantity adjuster adjusts the light quantity such that gray scale values of substantially all the pixels of the digital image exceed a predetermined threshold. The pill counting device thus can count pills in a dose packet having strings and/or drawing patterns (information identifiers) printed or handwritten thereon at high accuracy, by removing the image of the information identifiers alone through the adjustment of the quantity of light received by the imager. Unlike the system in PTL 1,the pill counting device only needs to adjust the quantity of the received light once for each type of dose packets, rather than the subtraction of an image every counting, and can thereby be operated under reduced load.

The adjustment of the quantity of the received light involves adjusting the luminance of the near-infrared light emitted from the illuminator. In other words, the adjustment of the luminance of the near-infrared light emitted from the illuminator, i.e., the adjustment of the brightness of the illuminator can control the quantity of the received light. The pill counting device thus has simple mechanisms and structures and can be produced at low costs.

The adjustment of the quantity of the received light involves adjusting the exposure time of the imager. For example, the adjustment of a shutter speed of the imager to adjust the exposure time of the imager can control the quantity of the received light. This configuration can enhance the usability of the pill counting device.

### Brief Description of Drawings

FIG. 1 is a plan view of an exemplary dose packet according to an embodiment of the invention;
FIG. 2 is a partial cross-sectional view of an exemplary pill counting device according to the embodiment of the invention;
FIG. 3 is a conceptual diagram to explain the reason for utilizing near-infrared light to be emitted from an illuminator according to the embodiment of the invention;
FIG. 4 is a flowchart of an exemplary process executed in a pill counting device according to the embodiment of the invention; and
FIG. 5 is a diagram illustrating an exemplary display screen of a pill counting device according to the embodiment of the invention.

### Description of Embodiments

A pill counting device according to an embodiment of the invention to count pills in a dose packet will now be described with reference to the drawings.

In the description below, the term "pills" indicates tablets containing compressed effective ingredients, capsules containing powdered medicines, and sugar-coated tablets.

The term "near-infrared light" indicates light having a wavelength of approximately 0.8 to 2.5 µm in the description below.

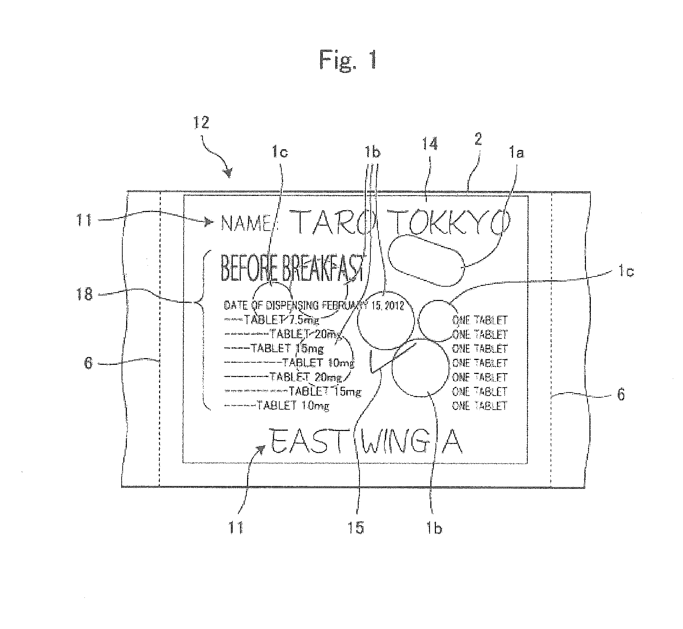

The term "information identifiers" indicates strings, drawing patterns, and marks printed or handwritten on a dose packet in the description below (refer to FIG. 1).

The following description focuses on a monochrome image for descriptive purposes as a typical example; hence, the gray scale value of each pixel of the image is indicated by any one of gray scales 0 to 255 in the gray scale system, for example.

In advance of the description of a pill counting device 10 according to the embodiment of the invention, a dose packet 12 and information identifiers according to the embodiment will now be described.

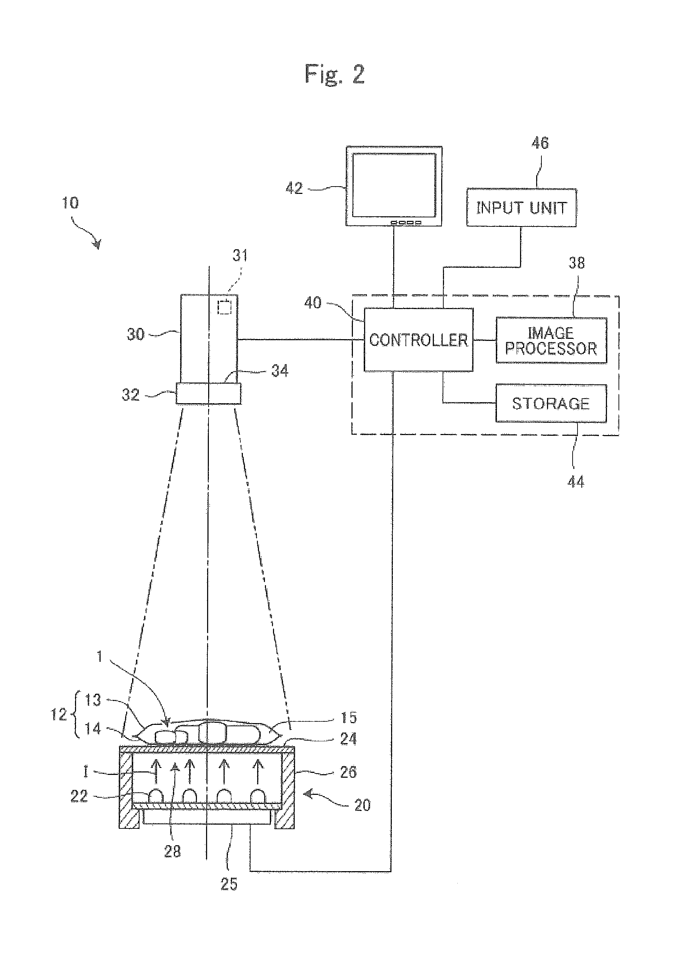

With reference to FIG. 1, the dose packet 12 according to the embodiment is a known package used in general hospitals and dispensing pharmacies to enclose pills. With reference to FIGS. 1 and 2, the dose packet 12 is one of the linearly continuous packets fabricated by folding a sheet strip in two along the longitudinal direction to form a folded edge 2 (refer to FIG. 1) and thermally welding upper and lower sheets 13 and 14 together at predetermined intervals in the longitudinal direction, for example. The thermally welded portions orthogonal to the longitudinal direction have perforated tear-off lines 6 to separate the continuous dose packets 12 from one another, as illustrated in FIG. 1.

The resulting dose packet 12 contains pills packed in accordance with a prescription with a known pill packaging machine (not shown), for example. The side of the dose packet 12 opposite to the folded edge 2 is open or unwelded to accept the pills, and is closed by welding after the reception of the pills.

The sheet strip is composed of a transparent or translucent synthetic resin, such as polyethylene, polypropylene, or poly(ethylene terephthalate), that can transmit near-infrared light.

The resulting dose packet 12 contains pills 1, such as a capsule 1a, tablets 1b, and sugar-coated tablets 1c, as illustrated in FIG. 1.

With reference to FIG. 1, the information identifiers according to the embodiment include printed strings 18, handwritten strings 11, such as "Mr. Taro Tokkyo (the name of a patient)" and "East-A (the name of a hospital ward)," and handwritten marks 15, such as ticks. The information identifiers may be printed or handwritten with an ink having a high transmittance of near-infrared light or an ink barely transmitting near-infrared light. The information identifiers may have various colors, such as blue, green, and red, or may have a black color alone.

According to the embodiment, the information identifiers are provided on either one (the sheet 14 in this case) of the upper and lower sheets 13 and 14.

The pill counting device 10 according to the embodiment will now be described. With reference to FIG. 2, the pill counting device 10 according to the embodiment includes an illuminator 20, an imager 30, a controller 40, an input unit 46, an image processor 38, a storage 44, and a display 42. FIG. 2 includes a partial cross-sectional view that describes the internal structure of the illuminator 20.

The controller 40 according to the embodiment includes a CPU (not shown) and a working memory (not shown). With reference to FIG. 2, the controller 40 is connected to the imager 30, the image processor 38, and the illuminator 20. The controller 40 controls the imaging operation of the imager 30, the image processing operation of the image processor 38, the luminance of the illuminator 20, and the writing operation of the storage 44, under the instructions from a predetermined program or the input unit 46, for example.

With reference to FIG. 2, the illuminator 20 according to the embodiment is a direct-lit backlight including multiple light sources 22, a frame 26, a diffuser plate 24, and a luminance adjuster 25.

The frame 26 has a shape of rectangular parallelepiped box having an open top 28, as illustrated in FIG. 2.

The light sources 22 are LED lamps emitting near-infrared light having a peak wavelength of 0.85 µm, for example. The light sources 22 are disposed on the bottom of the frame 26 and emit near-infrared light I toward the top 28, as illustrated in FIG. 2. The light sources 22 may be semiconductor lasers, for example, in place of the LED lamps.

The diffuser plate 24 consists of a substantially-flat thin plate to transmit and diffuse the light emitted from the light sources 22. The diffuser plate 24 covers the top 28 of the frame 26. The diffuser plate 24 may be composed of any material; but should preferably be composed of a material having a high diffusivity of the transmitted light, in specific, a whitish (translucent white) resin, such as a polycarbonate resin, acrylic resin, or polypropylene resin.

The luminance adjuster 25 includes a known control circuit to control current through the LED lamps (light sources 22), for example. The luminance adjuster 25 controls the current through the LED lamps under the instruction from the controller 40, to adjust the luminance of the near-infrared light emitted from the light sources 22. In other words, the luminance adjuster 25 can adjust the brightness of the light sources 22. The luminance adjuster 25 according to the embodiment further includes a knob (not shown) to vary the current.

The current may be controlled by varying the magnitude of the current or modulating the width of the pulsed current.

The illuminator 20 emits the near-infrared light I toward the sheet surface. With reference to FIG. 2, the dose packet 12 is disposed on the diffuser plate 24 placed substantially horizontally, such that the illuminator 20 emits the near-infrared light I toward the sheet 14 from substantially directly below the dose packet 12.

According to the embodiment, the dose packet 12 is disposed directly on the diffuser plate 24. In an alternative embodiment, the dose packet 12 is placed on a conveyer belt composed of a material that can transmit near-infrared light and horizontally moving, and the illuminator 20 is disposed at a predetermined position below the conveyor belt, for example.

For example, the imager 30 according to the embodiment includes a digital camera including an image sensor (not shown), such as a CCD image sensor or CMOS image sensor, and an exposure time adjuster 31 to adjust the exposure time such as a shutter speed. The image sensor should preferably have high sensitivity to near-infrared light.

With reference to FIG. 2, the imager 30 is provided with a visible-light blocking filter 32 at a light-receiving end 34 of the imager 30. The visible-light blocking filter 32 according to the embodiment is an optical filter that attenuates not near-infrared light but light in the visible range (e.g., 0.36 to 0.8 µm), to prevent the visible light from entering the image sensor.

The imager 30 is disposed at a position for receiving the near-infrared light I transmitted through the dose packet 12 to take an image of the dose packet 12. In specific, the imager 30 is disposed above the illuminator 20 so as to face the illuminator 20 as illustrated in FIG. 2, receives the near-infrared light I transmitted through the dose packet 12, and takes the image of the dose packet 12.

The imager 30 is disposed at any position depending on the orientation of the illuminator 20 and the attitude of the dose packet 12 other than the position above the illuminator 20, provided that the imager 30 can receive the near-infrared light transmitted through the dose packet 12. The image taken by the imager 30 will be described later.

The image processor 38 includes a CPU (not shown) and an image memory (not shown). The image processor 38 binarizes the gray scale value of each pixel of the original image taken by the imager 30 with a predetermined threshold through a known binarization process, for example. The binarization process produces a monochrome image, i.e., a binary image of the dose packet 12. The image processor 38 removes isolated pixels from the binary image through a known process for noise removal, for example.

For example, the input unit 46 includes a numeric keypad, a keyboard, and a mouse and is connected to the controller 40. The input unit 46 inputs characters and numerals into the controller 40. The storage 44 includes a known hard disk to store image data and data on the threshold for the binarization process.

The display 42 is a known liquid crystal display to display the original image and the binary image of the dose packet 12 and a graphical user interface (GUI) screen for the manipulation, under the instruction from the controller 40. The GUI screen includes various areas, windows, and buttons for the input through the keyboard and the manipulation of the mouse, as will be described later. The display 42 may include a touch panel to display the original image and the binary image of the dose packet 12 and the graphical user interface (GUI) screen for the manipulation.

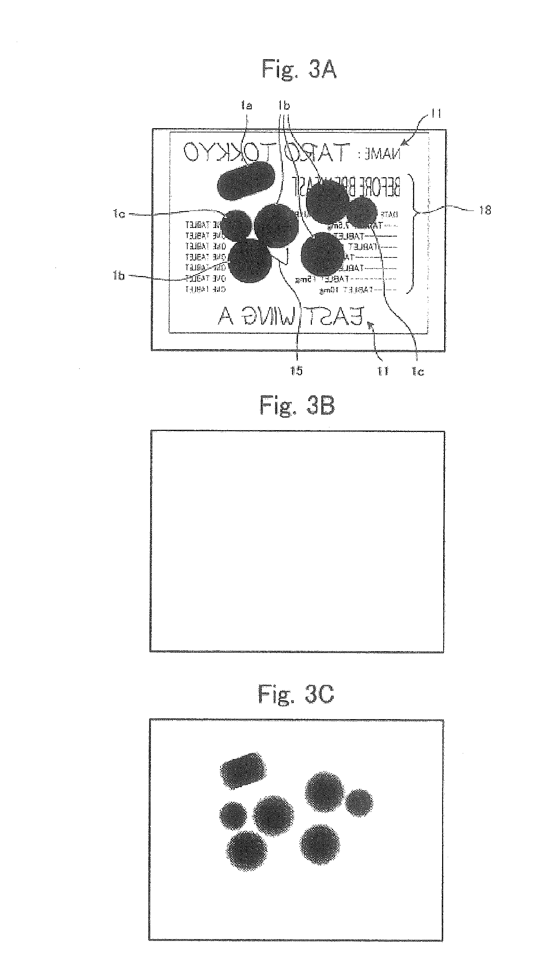

The reason for utilizing the near-infrared light to be emitted from the illuminator 20 will now be explained with reference mainly to FIG. 3.

When the pills in the dose packet 12 are counted on the basis of a digital image that captures the light transmitted through the dose packet 12, the information identifiers such as strings on the surface of the dose packet 12 disturb the counting operation. The present inventors have intensively studied the problematic phenomenon, focusing on the wavelength of the light and the quantity of the light received by the imager. The study has revealed that in the digital image capturing visible light, the gray scale values of the pixels for both the strings and pills similarly increase in accordance with an increase in the quantity of the received light; whereas in the digital image capturing near-infrared light, the gray scale values of the pixels for the strings increase earlier than those of the pills. In light of these phenomena, the inventors have accomplished the invention.

The adjustment of the quantity of the received light through varying the luminance at a constant exposure time will now be specifically described.

If the illuminator emits visible light at a low luminance, the gray scale values of the pixels for both the strings and pills are lower than those of the background in the image of the dose packet, such that the contrast of the strings and pills from the background can be observed as illustrated in FIG. 3A. As the luminance increases to raise the quantity of the visible light received by the imager, the gray scale values of the pixels for both the strings and pills similarly increase to levels comparable with those of the background, so that the contrast disappears from the image as illustrated in FIG. 3B. It is noted that FIG. 3A illustrates the front-back reversed image of the dose packet 12.

If the illuminator emits near-infrared light at a low luminance, the gray scale values of the pixels for both the strings and pills are lower than those of the background in the image of the dose packet, such that the contrast of the strings and pills from the background can be observed as illustrated in FIG. 3A, just like the case of the visible light. As the luminance increases to raise the quantity of the near-infrared light received by the imager, the gray scale values of the pixels for the strings increase to levels comparable with those of the background whereas the gray scale values of the pixels for the pills barely increase, so that the silhouettes of the pills alone can be observed as illustrated in FIG. 3C, unlike the case of the visible light.

These phenomena seem to be caused by differences in the scattering and transmission characteristics due to the different wavelengths between the visible light (0.36 to 0.8 µm) and the near-infrared light (approximately 0.8 to 2.5 µm).

For example, the visible light, which has a shorter wavelength than that of the near-infrared light, barely travels through an object but more readily scatters than the near-infrared light. The scattered visible light arrives above both the strings having a smaller thickness consisting mainly of the thickness of ink and the pills having larger thicknesses than that of the strings. This phenomenon increases the quantity of the light received by the light-receiving portions of the image sensor corresponding to the strings and pills, so that the contrast disappears from the image.

The transmitted or scattered near-infrared light readily arrives above the strings having a smaller thickness, so that the light-receiving portions of the image sensor corresponding to the strings receive an increased quantity of light. In contrast, the near-infrared light barely travels or scatters above the pills having larger thicknesses than that of the strings, so that the light-receiving portions of the image sensor corresponding to the pills receive a small quantity of light. The silhouettes of the pills alone can therefore be observed.

As the wavelength of the near-infrared light approaches the upper limit (2.5 µm), the difference observed in the images between the near-infrared light and the visible light tends to increase.

The same results are provided by the adjustment of the quantity of the light received by the imager through varying the exposure time at a constant luminance.

If the visible light from the illuminator is received during a short exposure time, the gray scale values of the pixels for both the strings and pills are lower than those of the background in the image of the dose packet, such that the contrast of the strings and pills from the background can be observed as illustrated in FIG. 3A. As the exposure time increases to raise the quantity of the received visible light, the gray scale values of the pixels for both the strings and pills similarly increase to levels comparable with those of the background, so that the contrast disappears from the image as illustrated in FIG. 3B.

If the near-infrared light from the illuminator is received during a short exposure time, the gray scale values of the pixels for both the strings and pills are lower than those of the background in the image of the dose packet, such that the contrast of the strings and pills from the background can be observed as illustrated in FIG. 3A, just like the case of the visible light. As the exposure time increases to raise the quantity of the received near-infrared light, the gray scale values of the pixels for the strings increase to levels comparable with those of the background whereas the gray scale values of the pixels for the pills barely increase, so that the silhouettes of the pills alone can be observed as illustrated in FIG. 3C, unlike the case of the visible light. In light of these phenomena, the inventors have accomplished the invention.

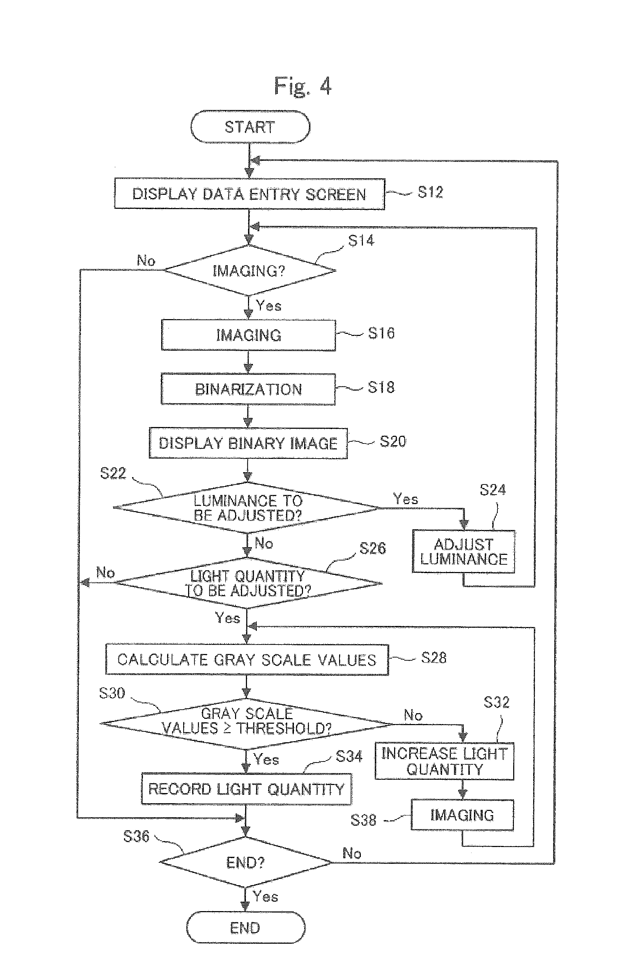

An exemplary operation of the pill counting device 10 according to the embodiment will now be explained with reference to FIG. 4. FIG. 4 illustrates an exemplary flow of the process executed in the pill counting device 10 according to the embodiment.

### [Exemplary Process Flow]

In Step S12, the controller 40 controls the display 42 to display a data entry screen 41 (GUI screen described above).

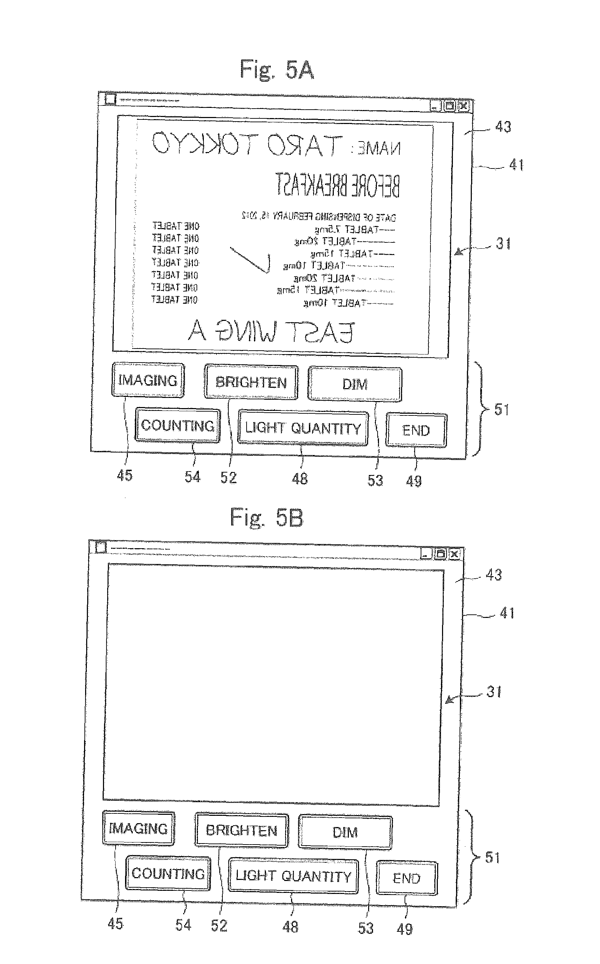

An exemplary data entry screen according to the embodiment will now be described with reference to FIG. 5A. For example, the data entry screen 41 according to the embodiment includes a dose-packet image display area 43 and an operation instructing area 51 as illustrated in FIG 5A. The dose-packet image display area 43 displays an original image or a binary image of the dose packet 12. With reference to FIG. 5A, the operation instructing area 51 displays, for example, an imaging button 45 to request the imaging, a brighten button 52 to increase the luminance, a dim button 53 to decrease the luminance, a light quantity button 48 to request the adjustment of the quantity of the received light, an end button 49 to terminate the process, and a counting button 54 to request the counting of pills. An operator can input instructions to the controller 40 through the data entry screen 41.

The explanation of the process flow will be resumed. With reference to FIG. 2, the operator disposes an empty dose packet 12 containing no pill above the illuminator 20, in more specific, on the diffuser plate 24, such that the lower sheet 14 having strings thereon faces downward.

In Step S 14, the controller 40 determines whether the imaging button 45 is clicked. If a click is determined; then the process goes to Step S16; otherwise the process goes to Step S36.

In Step S16, the controller 40 instructs the illuminator 20 to emit near-infrared light and instructs the imager 30 to take an image. Under the instruction, the imager 30 acquires original image data on the dose packet 12 and transmits the original image data to the image processor 38. The process of the controller 40 then proceeds to Step S18.

In Step S18, the controller 40 instructs the image processor 38 to binarize the original image data. Under the instruction, the image processor 38 binarizes the original image data with a first threshold (e.g., 130 gray level) to generate a binary image. The controller 40 then instructs the image processor 38 to remove the noise from the binary image. Under the instruction, the image processor 38 removes the noise from the binary image, and writes the information on the coordinates of isolated pixels and the binary image data after the noise removal into the storage 44.

The process of the controller 40 then proceeds to Step S20.

In Step S20, the controller 40 controls the display 42 to display the binary image of the dose packet 12 (refer to FIG. 5A) on the basis of the binary image data.

If the operator cannot observe the strings on the dose packet 12 on the display 42, the operator can adjust the luminance of the illuminator 20 to make the strings on the dose packet 12 visible by clicking the brighten button 52 or the dim button 53 in the operation instructing area 51.

In other words, in Step S22, the controller 40 determines whether the dim button 53 is clicked. If the dim button 53 is clicked; then the process proceeds to Step S24; otherwise the controller 40 determines whether the brighten button 52 is clicked. If the brighten button 52 is clicked; then the process of the controller 40 proceeds to Step S24; otherwise the process proceeds to Step S26.

In Step S24, the controller 40 instructs the luminance adjuster 25 to adjust the luminance. In specific, the controller 40 instructs the luminance adjuster 25 to decrease the luminance by a predetermined value if the dim button 53 is clicked, and instructs the luminance adjuster 25 to increase the luminance by a predetermined value if the brighten button 52 is clicked.

The operator thus can adjust the luminance of the illuminator 20 while watching the display 42, such that the strings on the dose packet 12 are visible on the screen (refer to FIG. 5A).

In Step S26, the controller 40 determines whether the light quantity button 48 is clicked. If a click is determined; then the process goes to Step S28; otherwise the process goes to Step S36.

In Step S28, the controller 40 calculates the gray scale values of substantially all the pixels of the original image transmitted to the image processor 38, after the removal of the isolated pixels from all the pixels through the noise removal. For example, the controller 40 generates a table of the gray scale values of the respective pixels and records the table into the storage 44. The process then proceeds to Step S30.

In Step S30, the controller 40 compares the gray scale values of substantially all the pixels to a second threshold (e.g., 240 gray level). If the gray scale values are equal to or larger than the second threshold; the process goes to Step S34; otherwise the process goes to Step S32.

In Step S32, the controller 40 instructs the luminance adjuster 25 to increase the luminance by a predetermined value. The process then goes to Step S38.

In Step S38, the controller 40 instructs the illuminator 20 to emit near-infrared light and instructs the imager 30 to take an image at a constant exposure time. Under the instruction, the imager 30 acquires original image data on the dose packet 12 and transmits the original image data to the image processor 38. The process of the controller 40 then proceeds to Step S28.

In Step S34, the controller 40 controls the storage 44 to record the adjusted luminance set in the luminance adjuster 25 and the exposure time. The process then goes to Step S36. FIG. 5B illustrates the image after the removal of the information identifiers (e.g., strings and marks) through the adjustment of the quantity of the received light.

In Step S36, the controller 40 determines whether the end button 49 is clicked. If a click is determined; then the process is terminated; otherwise the process goes to Step S12.

As is explained mainly in Steps S26 to S38, the controller 40 functions as a light quantity adjuster to adjust the quantity of the near-infrared light received by the imager 30 during the exposure. For a digital image of the dose packet 12 containing no pill in which the gray scale values of the pixels for the information identifiers are lower than the first threshold and those of the background of the information identifiers are equal to or higher than the first threshold, the quantity of the received light is adjusted such that the gray scale values of substantially all the pixels exceed the second threshold higher than the first threshold.

As is explained in Step S32, the adjustment of the quantity of the received light according to the embodiment involves the control of the luminance of the illuminator 20.

In the pill counting device 10 according to the embodiment, the controller 40 functions as the light quantity adjuster. In place of the controller 40, the image processor 38 including the CPU may function as the light quantity adjuster.

The quantity of the received light is adjusted through varying the luminance at a constant exposure time in the above process flow. Alternatively, the quantity of the received light may be adjusted through any process, such as through varying the exposure time at a constant luminance.

For example, in Step S32, the controller 40 may instruct the imager 30 to increase the exposure time by a predetermined period without varying the luminance set in the luminance adjuster 25, and then to proceed to Step S38. In other words, the adjustment of the quantity of the received light may involve the control of the exposure time of the imager.

### [Counting Pills]

The pills enclosed in the dose packet 12 are counted while the near-infrared light having the luminance adjusted through the above process flow is being emitted toward the dose packet 12. In specific, the controller 40 instructs the illuminator 20 to emit near-infrared light at the adjusted luminance, for example, in response to a click on the counting button 54. The controller 40 then instructs the imager 30 to take a digital image of the dose packet 12 through the reception of the near-infrared light during the exposure time and to transmit the digital image to the image processor 38. The image processor 38 receives the digital image, and binarizes the digital image with the second threshold to generate a monochrome image (refer to FIG. 3C) including the silhouettes of the pills alone. The image processor 38 then counts the pills through any image processing operation, such as labelling, on the monochrome image. That is, the controller 40 and the image processor 38 function as a pill counter that counts pills on the basis of the digital image taken by the imager 30.

The pill counting device 10 according to the embodiment thus can count the pills in the dose packet 12 having strings and/or drawing patterns printed or handwritten thereon at high accuracy under reduced processing load on the device 10.

The embodiments of the invention described in detail above with reference to the drawings are mere examples and may be variously modified or improved based on the technical knowledge of those skilled in the art. For example, the pill counting device may further acquire a silhouette image of each type of pills under predetermined imaging conditions and generate a corresponding table of the silhouette images and the types of pills in advance, to determine a type of pills in a dose packet through the comparison of the silhouette images of the pills to those stored in the corresponding table.

### Reference Signs List

- 1: pill
- 10: pill counting device
- 12: dose packet
- 13: upper sheet
- 14: lower sheet
- 11, 18: string (information identifier)
- 15: mark (information identifier)
- 20: illuminator
- 30: imager
- 38: image processor (pill counter)
- 40: controller (light quantity adjuster, pill counter)
- I: near-infrared light

## Claims

1. A pill counting device (10)for counting pills(1) in a dose packet(12) including two near-infrared light transmissive sheets(13,14) laminated and sealed at the periphery, a first sheet (14)of the sheets having an information identifier(11,15,18) thereon, the device comprising:
an illuminator(20) to emit near-infrared light toward the dose packet(12) from below, the dose packet(12) being disposed such that the first sheet(14)faces downward;
an imager(30) disposed at a position to receive the near-infrared light transmitted through the dose packet(12), the imager(30) taking a digital image of the dose packet(12);
a light quantity adjuster(40) to adjust a quantity of the near-infrared light received by the imager(30); and
a pill counter(40,38)to binarize the digital image and count the pills(1), wherein
for a digital image of the dose packet(12) containing no pill, the light quantity adjuster(40) adjusts the light quantity such that gray scale values of substantially all the pixels of the digital image exceed a predetermined threshold.

2. The pill counting device(10) according to claim 1, wherein the adjustment of the light quantity involves adjusting a luminance of the near-infrared light emitted from the illuminator(20).

3. The pill counting device(10) according to claim 1 or 2, wherein the adjustment of the light quantity involves adjusting an exposure time of the imager(30).
